# EUROPÄISCHE PATENTANMELDUNG

(11) **EP 4 548 947 A1**
(43) Veröffentlichungstag der Anmeldung: **07.05.2025**
(21) Anmeldenummer: 23207090.4
(22) Anmeldetag: 31.10.2023
(51) Int. Cl.: A61M 5/20, A61M 5/32

(54) **VORRICHTUNG ZUM VERABREICHEN EINER FLUIDEN SUBSTANZ**

(71) Anmelder: Ypsomed AG, 3401 Burgdorf (CH)
(72) Erfinder: Loser, Peter, 3037 Herrenschwanden (CH); Tschirren, Markus, 3400 Burgdorf (CH); Schrul, Christian, 3400 Burgdorf (CH)
(74) Vertreter: Eugster, Monika Katharina

(57) **Zusammenfassung**

Die Erfindung betrifft eine Vorrichtung zum Verabreichen einer fluiden Substanz. Die erfindungsgemässe Vorrichtung umfasst ein Gehäuse (1), ein gehäusefestes Halteelement (6; 6'), in welchem eine Injektionsfeder (5) aufgenommen ist. Das Halteelement (6; 6') kann zwischen einer Eingriffsposition, in welcher das Halteelement (6; 6') in Eingriff mit der Injektionsfeder (5) ist, und einer Auslöseposition, in welcher das Halteelement (6; 6') ausser Eingriff mit der Injektionsfeder (5) ist, radial nach aussen bewegbar oder radial auslenkbar sein. Bei einer axialen Bewegung eines Nadelschutzelements (3) von der distalen Position in die proximale Position kann das Halteelement (6; 6') ausser Eingriff mit der Injektionsfeder (5) gelangen, um die Substanz aus dem Produktbehälter (2) durch die Nadel (2b) zu verabreichen.

## Beschreibung

Die Erfindung betrifft eine Vorrichtung mit einer Längsachse (L) zum Verabreichen einer fluiden Substanz, insbesondere eines Medikaments.

Der Begriff «Medikament» umfasst hier jede fliessfähige medizinische Formulierung, welche geeignet ist zur kontrollierten Verabreichung durch ein Mittel wie eine Kanüle oder Nadel hindurch, beispielsweise umfassend eine Flüssigkeit, eine Lösung, ein Gel, oder eine feine Suspension, welche einen oder mehrere medizinische Wirkstoffe enthält. Medikament kann eine Zusammensetzung mit einem einzigen Wirkstoff oder eine vorgemischte oder co-formulierte Zusammensetzung mit mehreren Wirkstoffen aus einem einzelnen Behälter sein. Medikament umfasst Arzneien wie Peptide (z.B. Insuline, Insulin enthaltende Medikamente, GLP-1 enthaltende sowie abgeleitete oder analoge Zubereitungen), Proteine und Hormone, biologisch gewonnene oder aktive Wirkstoffe, Wirkstoffe auf Basis von Hormonen oder Genen, Nährformulierungen, Enzymen und weitere Substanzen sowohl in fester (suspendierter) oder flüssiger Form aber auch Polysaccaride, Vaccine, DNS oder RNS oder Oglionukletoide, Antikörper oder Teile von Antikörpern sowie geeignete Basis-, Hilfs- und Trägerstoffe.

Aus dem Stand der Technik sind Vorrichtungen zum Verabreichen einer fluiden Substanz bekannt. Aus der EP2742962B1 ist beispielsweise ein Autoinjektor mit einem axial verschiebbaren Halteelement bekannt, wobei das Halteelement ausser Eingriff mit einer Kolbenstange gelangen kann. Zudem ist die mit einer Injektionsfeder aufgenommene Kolbenstange in die distale Richtung bewegbar, um auf einen Stopfen einer Spritze zu wirken, sodass eine fluide Substanz verabreicht wird.

Nachteilig bei diesem Autoinjektor ist, dass dieser Autoinjektor komplex aufgebaut ist und viele Bestandteile aufweist.

Es ist eine Aufgabe der Erfindung, eine Vorrichtung zum Verabreichen einer fluiden Substanz bereitzustellen, welche einfach und mit wenigen Bestandteilen aufgebaut ist.

Im Folgenden bedeutet die proximale Richtung bei einer Vorrichtung zum Verabreichen einer fluiden Substanz die Richtung zum gehäuseseitigen Ende hin und die distale Richtung bei dieser Vorrichtung die Richtung zum nadelseitigen oder kanülenseitigen Ende hin.

Die erfindungsgemässe Vorrichtung mit einer Längsachse (L) zum Verabreichen einer fluiden Substanz, insbesondere eines Medikaments weist ein Gehäuse auf. Das Gehäuse ist bevorzugt länglich ausgebildet. Das Gehäuse kann bevorzugt hülsenförmig und/oder zylindrisch, insbesondere kreiszylindrisch ausgebildet sein. In dem Gehäuse ist ein Produktbehälter axial fest aufgenommen. Der Produktbehälter umfasst die zu verabreichende Substanz. Ferner ist in dem Produktbehälter ein Stopfen vorgesehen, welcher axial verschiebbar in dem Produktbehälter aufgenommen ist. Zudem weist der Produktbehälter eine Nadel oder Kanüle auf, durch welche die Substanz verabreicht werden kann. Der Stopfen ist bevorzugt von einem proximalen Ende des Produktbehälters zu einem distalen Ende des Produktbehälters in dem Produktbehälter axial verschiebbar angeordnet, um die in dem Produktbehälter vorgesehene Substanz zu verabreichen. Der Produktbehälter kann als Spritze, welche einen Spritzenkörper und eine am distalen Ende des Spritzenkörpers fest oder unlösbar aufgenommene Nadel oder Kanüle umfassen. Alternativ kann der Produktbehälter als Karpule mit einem Karpulenkörper und einer aufsetzbaren und wegnehmbaren Nadeleinheit ausgebildet sein.

Des Weiteren umfasst die erfindungsgemässe Vorrichtung ein relativ zu dem Gehäuse verschiebbares Nadelschutzelement für die Nadel oder Kanüle. Das Nadelschutzelement kann hülsenförmig und/oder zylindrisch, insbesondere kreiszylindrisch ausgebildet sein. Das Nadelschutzelement kann zum Schutz vor Stichverletzungen durch die Nadel oder durch die Kanüle, insbesondere durch eine distale Spitze der Nadel oder durch eine distale Spitze der Kanüle dienen. Ferner weist die Vorrichtung eine Nadelschutzfeder auf, welche mit dem Nadelschutzelement gekoppelt ist, wobei das Nadelschutzelement von einer distalen Position, in welcher die distale Spitze der Nadel oder der Kanüle von dem Nadelschutzelement umgeben ist und in eine proximale Position, in welcher die distale Spitze der Nadel oder der Kanüle von dem Nadelschutzelement freigeben ist, axial bewegbar ist.

Ferner weist die erfindungsgemässe Vorrichtung eine Injektionsfeder auf, welche die Energie zur Bewirkung der Substanzverabreichung zur Verfügung stellt und welche mit dem Stopfen zusammenwirkt, um die Substanz aus dem Produktbehälter durch die Nadel oder durch die Kanüle zu verabreichen. Die Vorrichtung kann als Autoinjektor ausgebildet sein. Autoinjektoren werden oft als Vorrichtungen bezeichnet, welche eine zu verabreichende Substanz nach der Auslösung automatisch einem Patienten abgeben.

Die Nadelschutzfeder und die Injektionsfeder können als Druckfeder ausgebildet sein. Besonders bevorzugt sind die beiden Federn als Wendelfeder, insbesondere als Wendelfeder mit einer oder mehreren Windungen ausgebildet. Es können aber auch andere Federn vorgesehen sein. Die Nadelschutzfeder und die Injektionsfeder sind bevorzugt vorgespannt in der erfindungsgemässen Vorrichtung vorgesehen. Die Nadelschutzfeder und die Injektionsfeder sind spannbar und entspannbar ausgebildet. Die Kraft der Nadelschutzfeder bewirkt, dass die Nadelschutzfeder das Nadelschutzelement von der distalen Position in die proximale Position und wieder in die distale Position oder in eine von der distalen Position unterschiedliche distale Position verschoben werden kann. Die Kraft der Injektionsfeder bewirkt, dass die Injektionsfeder die gesamte Menge der zu verabreichenden Substanz oder eine Teilmenge der zu verabreichenden Substanz ausschütten kann.

Die erfindungsgemässe Vorrichtung umfasst zudem ein relativ zu dem Gehäuse axial fest angeordnetes Halteelement, in welchem die Injektionsfeder, insbesondere vorgespannt und entspannbar aufgenommen ist. Das Halteelement ist zwischen einer Eingriffsposition, in welcher das Halteelement in Eingriff mit der Injektionsfeder ist, und einer Auslöseposition, in welcher das Halteelement ausser Eingriff mit der Injektionsfeder ist, radial nach aussen bewegbar oder radial auslenkbar. Das Halteelement ist bevorzugt länglich ausgebildet und erstreckt sich vorzugsweise entlang der Längsachse (L). Besonders bevorzugt umfasst das Halteelement einen Haltearm, welcher radial nach aussen bewegbar oder radial auslenkbar ist. Es kann eine Vielzahl von Haltearmen vorgesehen sein. Der Haltearm des Halteelements erstreckt sich bevorzugt von einem proximalen Ende zu einem distalen Ende, wobei das proximale Ende des Haltearms bevorzugt axial fest an dem Gehäuse angeordnet ist. Das Halteelement, insbesondere der Haltearm des Halteelements ist bevorzugt elastisch ausgebildet. Das Halteelement, insbesondere der Haltearm des Halteelements ist bevorzugt derart elastisch ausgebildet, dass es, insbesondere er zwischen einer Eingriffsposition, in welcher es, insbesondere er in Eingriff mit der Injektionsfeder, insbesondere mit der oder den Windungen der Injektionsfeder ist, und einer Auslöseposition, in welcher es, insbesondere er ausser Eingriff mit der Injektionsfeder, insbesondere mit der oder den Windungen der Injektionsfeder ist, radial nach aussen bewegbar oder radial auslenkbar ist. Das Halteelement, insbesondere der Haltearm des Halteelements kann eine Haltenocke aufweisen. Die Haltenocke kann derart ausgebildet sein, dass sie in Eingriff und ausser Eingriff mit Injektionsfeder, insbesondere in Eingriff und ausser Eingriff mit der oder den Windungen der Injektionsfeder gelangbar ist. Das Halteelement, insbesondere der Haltearm des Halteelements oder die Haltenocke des Halteelements ist derart ausgebildet, dass die Injektionsfeder, insbesondere die Windung oder die Windungen der Injektionsfeder vorgespannt in Eingriff gehalten werden kann.

Zudem gelangt bei der axialen Bewegung des Nadelschutzelements von der distalen Position in die proximale Position das Halteelement, insbesondere der Haltearm des Halteelements, besonders bevorzugt die Haltenocke des Haltearms des Halteelements ausser Eingriff mit der Injektionsfeder, um die Substanz aus dem Produktbehälter durch die Nadel oder durch die Kanüle zu verabreichen. Das Nadelschutzelement kann derart ausgebildet sein, dass bei der axialen Bewegung des Nadelschutzelements von der distalen Position in die proximale Position das Halteelement, insbesondere der Haltearm des Halteelements, besonders bevorzugt die Haltenocke des Haltearms des Halteelements ausser Eingriff mit der Injektionsfeder gelangt, um die Substanz aus dem Produktbehälter durch die Nadel oder durch die Kanüle zu verabreichen. Die in dem Halteelement vorgespannte Injektionsfeder kann ein radiales nach aussen Bewegen oder ein radiales Auslenken des Halteelements, insbesondere des Haltearms des Halteelements bewirken. Die Injektionsfeder kann sich ferner entspannen, wenn das Halteelement ausser Eingriff mit der Injektionsfeder ist, wodurch die Substanz aus dem Produktbehälter durch die Nadel oder durch die Kanüle verabreicht werden kann. Der Vorteil der erfindungsgemässen Vorrichtung ist eine kompakte Grösse, insbesondere der Aussenmasse der Vorrichtung. Die erfindungsgemässe Vorrichtung ist derart optimiert, dass sie wenige Bestandteile aufweist. Zudem ist die erfindungsgemässe Vorrichtung einfach und robust ausgebildet.

Das Halteelement kann in anderen Ausführungsformen eine andere Gestalt aufweisen, wobei das Halteelement derart ausgebildet ist, dass es relativ zu dem Gehäuse der Vorrichtung axial fest angeordnet ist und zwischen der Eingriffsposition, in welcher das Halteelement in Eingriff mit der Injektionsfeder ist, und einer Auslöseposition, in welcher das Haltelement ausser Eingriff mit der Injektionsfeder ist, radial nach aussen bewegbar oder radial auslenkbar ist. Das Halteelement kann in der Eingriffsposition die Injektionsfeder vorgespannt in Eingriff halten. Ferner kann sich in der Auslöseposition die Injektionsfeder entspannen, wodurch die Substanz aus dem Produktbehälter verabreicht werden kann.

Um die Substanz aus dem Produktbehälter zu verabreichen, kann die Injektionsfeder in einer Ausführungsform der erfindungsgemässen Vorrichtung unmittelbar auf den Stopfen in dem Produktbehälter wirken. Diese Ausführungsform der erfindungsgemässen Vorrichtung hat den Vorteil, dass dadurch die Vorrichtung eine hohe Verabreichungsleistung und eine kompakte Bauweise aufweist. In einer alternativen Ausführungsform der erfindungsgemässen Vorrichtung ist zwischen der Injektionsfeder und dem Stopfen ein Adapter vorgesehen, um die Substanz zu verabreichen. Die Injektionsfeder wirkt somit über den Adapter auf den Stopfen, um die Substanz zu verabreichen. Diese Ausführungsform der erfindungsgemässen Vorrichtung hat den Vorteil, dass dadurch verschieden geformte Injektionsfedern und verschieden geformte Stopfen zusammengebaut werden können und trotzdem eine Vorrichtung mit einer hohen Verabreichungsleistung und einer kompakten Bauweise bereitgestellt werden kann. Der Adapter kann länglich ausgebildet sein und sich entlang der Längsachse (L) erstrecken. Der Adapter kann hülsenförmig oder als Vollmaterial ausgebildet sein. In einer Ausführungsform der erfindungsgemässen Vorrichtung kann das distale und das proximale Ende des Adapters derart ausgebildet sein, dass die Enden einerseits in einen Anschlagkontakt mit dem Stopfen des Produktbehälters und andererseits in einen Anschlagkontakt mit der Injektionsfeder gelangen können. Der Adapter kann zudem als Dämpfungselement ausgebildet sein, um die Kraft der Injektionsfeder zu verringern oder entsprechend anzupassen. Der Adapter kann ferner eine Führung zur Führung der Injektionsfeder aufweisen. Die Führung des Adapters kann an einer Aussenfläche des Adapters vorgesehen sein. Dadurch kein ein Verbiegen der Injektionsfeder verhindert werden. In einer Ausführungsform der erfindungsgemässen Vorrichtung kann das distale Ende der Injektionsfeder an einem distalen Teil des Adapters abgestützt sein und das proximale Ende der Injektionsfeder kann an einem proximalen Teil des Gehäuses oder an einem proximalen Teil des Halteelements abgestützt sein. Bei der erfindungsgemässen Vorrichtung entspannt sich die Injektionsfeder während der Verabreichung der fluiden Substanz unmittelbar in den Produktbehälter. Dadurch kann vorteilhaft die Bauweise der erfindungsgemässen Vorrichtung optimiert werden. Bei dieser Entspannung kann die Injektionsfeder auf den Stopfen oder alternativ auf den Adapter wirken, um die Substanz aus dem Produktbehälter zu verabreichen.

Der Begriff «unmittelbar» bedeutet in dieser Schrift «direkt» oder «durch keinen oder nur geringen Abstand voneinander getrennt». Beispielsweise ist in einer Ausführungsform der erfindungsgemässen Vorrichtung zwischen der Injektionsfeder und dem Stopfen kein Bestandteil (z.B. keine Kolbenstange) angeordnet. Die Injektionsfeder kann beispielsweise in dieser Ausführungsform der erfindungsgemässen Vorrichtung direkt auf den Stopfen wirken. Zudem ist beispielsweise zwischen der Injektionsfeder und dem Produktbehälter kein Bestandteil (z.B. eine Kolbenstange) angeordnet, wobei nur ein geringer Abstand oder kein Abstand zwischen der Injektionsfeder und dem Produktbehälter vorhanden ist. Der Begriff «unmittelbar» kann somit sowohl «entlang der Längsachse (L)» als auch «quer zur Längsachse (L)» gelesen werden.

Ferner kann beim Verabreichen der Substanz die Injektionsfeder, insbesondere die Windungen der Injektionsfeder relativ zu dem Halteelement und über das Halteelement entspannbar sein, um ein akustisches, visuelles und/oder taktiles Ausschüttsignal zu erzeugen. Das Ausschüttsignal kann durch eine relative Bewegung zwischen dem Halteelement, insbesondere dem Haltearm des Halteelements oder der Haltenocke des Halteelements und der Injektionsfeder, insbesondere der Windungen der Injektionsfeder erzeugt werden. Das akustische Signal kann beispielsweise ein hörbares Rattern oder Klicken, verursacht durch eine relative Bewegung zwischen dem Halteelements, insbesondere dem Haltearms des Halteelements oder der Haltenocke des Halteelements und den Windungen der Injektionsfeder während dem Verabreichen der Substanz sein. Zur Realisierung des visuellen Signals kann beispielsweise ein Fenster im Gehäuse der erfindungsgemässen Vorrichtung vorgesehen sein, durch welches die Entspannung der Windungen der Injektionsfeder ersichtlich ist. Das taktile Signal kann beispielsweise ein fühlbares Vibrieren sein, wenn sich die Windungen der Injektionsfeder relativ zu dem Halteelement, insbesondere dem Haltearm des Halteelements oder der Haltenocke des Halteelements entspannen.

In einer weiteren Ausführungsform der erfindungsgemässen Vorrichtung kann das Ausschüttsignal durch ein zusätzliches Halteelement, insbesondere einen zusätzlichen Haltearm des Halteelements oder eine zusätzliche Haltenocke des Halteelement erzeugt werden. Somit kann die erfindungsgemässe Vorrichtung ein erstes Halteelement und ein zweites Halteelement, insbesondere einen ersten Haltearm und einen zweiten Haltearm oder eine erste Haltenocke und eine zweite Haltenocke aufweisen. Das erste Halteelement, insbesondere der erste Haltearm oder die erste Haltenocke kann dazu dienen, um die Injektionsfeder in Eingriff oder ausser Eingriff zu halten und das zweite Halteelement, insbesondere der zweite Haltearm oder die zweite Haltenocke kann dazu dienen, das Ausschüttsignal zu erzeugen. Das zweite Halteelement, insbesondere der zweite Haltearm oder die zweite Haltenocke kann elastisch ausgebildet sein.

Bevorzugt ist das Halteelement oder der Haltearm des Halteelements oder die Haltenocke des Halteelements zur Erzeugung des Ausschüttsignals derart ausgebildet, dass die Frequenz des wahrnehmbaren Ausschüttsignals während dem Verabreichungsvorgang geringer wird, um dem Patienten anzuzeigen, dass die Verabreichung zu Ende geht. Dazu kann beispielsweise die Frequenz des hörbaren Ratterns oder Klickens während dem Verabreichen der Substanz, geringer werden. Ferner kann der ersichtliche Abstand zwischen den Windungen der Injektionsfeder während dem Verabreichen der Substanz grösser werden. Des Weiteren kann beispielsweise die Frequenz des fühlbaren Vibrierens während dem Verabreichen der Substanz geringer werden. Somit kann dem Patienten das kurz bevorstehende und/oder das Ende der Verabreichung der Substanz aus dem Produktbehälter angezeigt werden.

Die erfindungsgemässe Vorrichtung kann des Weiteren eine Schalthülse umfassen, welche entlang der Längsachse (L) zwischen der Nadelschutzfeder und dem Nadelschutzelement angeordnet ist, wobei die Schalthülse von dem Nadelschutzelement in die proximale Richtung mitgenommen wird, wenn das Nadelschutzelement aus der distalen Position in die proximale Position verschoben wird. Bevorzugt kann die Schalthülse, welche zwischen der Nadelschutzfeder und dem Nadelschutzelement angeordnet ist, von einer distalen Position in eine proximale Position und wieder in die distale Position oder in eine von der distalen Position unterschiedliche distale Position verschoben werden. Die Schalthülse ist bevorzugt länglich und hülsenförmig ausgebildet. Das Schalthülse ist bevorzugt zylindrisch, insbesondere kreiszylindrisch ausgebildet.

Die erfindungsgemässe Vorrichtung kann ferner eine Sperrhülse aufweisen, welche quer zur Längsachse (L) zwischen der Schalthülse und dem Haltelement angeordnet ist, wobei die Sperrhülse in der distalen Position des Nadelschutzelements ein radiales nach aussen Bewegen oder ein radiales Auslenken des Halteelements aus dem Eingriff zwischen dem Halteelement und der Injektionsfeder verhindert. Die Sperrhülse ist von einer distalen Position in eine proximale Position axial verschiebbar angeordnet. Das proximale Ende der Nadelschutzfeder kann an dem proximalen Ende der Sperrhülse abgestützt sein. Ferner kann die Sperrhülse axial bewegbar auf oder an dem Halteelement angeordnet sein. Die Sperrhülse kann in der distalen Position des Nadelschutzelements in einem lösbaren Eingriff mit dem Haltelement sein. Dazu kann beispielsweise die Sperrhülse einen Vorsprung aufweisen, welcher lösbar in Eingriff mit einer Ausnehmung an dem Haltelement sein kann. Alternativ kann der Vorsprung der Sperrhülse das distale Ende des Haltelements umgreifen und so einen lösbaren Eingriff bilden.

In der proximalen Position des Nadelschutzelements kann die Schalthülse die Sperrhülse freigeben und das Haltelement kann in die Auslöseposition mit der Injektionsfeder gelangen. Die Injektionsfeder wirkt derart auf das Halteelement, dass das Halteelement ausser Eingriff mit der Injektionsfeder radial nach aussen bewegt oder radial ausgelenkt wird. Die Sperrhülse gelangt ausser Eingriff mit dem Halteelement. Durch die beaufschlagende Kraft der Nadelschutzfeder kann somit die Sperrhülse von der distalen Position in die proximale Richtung axial verschiebt oder bewegt werden. Die Sperrhülse ist relativ zu dem gehäusefesten Halteelement in die proximale Richtung bewegbar und kann mit dem Haltelement in Anschlagkontakt gelangen, um ein akustisches, visuelles und/oder taktiles Ausschüttstartsignal zu erzeugen. Somit kann dem Patienten der Beginn der Verabreichung der Substanz aus dem Produktbehälter angezeigt werden. Das akustische Signal kann beispielsweise als hörbarer Anschlagkontakt zwischen der Sperrhülse und dem Haltelement ausgebildet sein. Zudem kann das Gehäuse der erfindungsgemässen Vorrichtung beispielsweise ein Fenster aufweisen, um die axiale Verschiebung der Sperrhülse relativ zu dem Halteelement sichtbar anzuzeigen. Während der axialen Bewegung zwischen der Sperrhülse und dem Halteelement kann ein fühlbares Vibrieren erzeugt werden, um ein taktiles Ausschüttsignal zu erzeugen. Die Sperrhülse ist bevorzugt länglich und hülsenförmig ausgebildet. Die Sperrhülse ist bevorzugt zylindrisch, insbesondere kreiszylindrisch ausgebildet.

Ferner können die Sperrhülse und die Schalthülse eine Kulissenführung, einen Sperranschlag und ein korrespondierendes spannbares Sperrelement aufweisen. Bevorzugt sind die Sperrhülse und die Schalthülse relativ zueinander axial bewegbar und relativ zueinander drehbar angeordnet. Der Sperranschlag kann rampenförmig ausgebildet sein, wobei das Sperrelement während einer Bewegung in distaler Richtung über die Rampe des Sperranschlags radial ausgelenkt werden kann und hinter einem Absatz des rampenförmigen Sperranschlags einschnappen kann. Das Sperrelement ist bevorzugt elastisch ausgebildet. Das Sperrelement kann als elastischer Sperrarm oder elastischer Sperrschnapper ausgebildet sein.

Bevorzugt kann während der Bewegung des Nadelschutzelements in die proximale Richtung durch die Kulissenführung zwischen der Sperrhülse und der Schalthülse die Sperrhülse und die Schalthülse relativ zueinander gedreht werden, wobei das Sperrelement in eine axiale Flucht zu dem Sperranschlag gelangen kann, um eine erneute Bewegung des Nadelschutzelements in die proximale Position zu verhindern. Somit ist das Nadelschutzelement gesperrt und kann den Patienten vor einer Verletzung durch die Nadel oder Kanüle, insbesondere durch die distale Spitze der Nadel oder durch die distale Spitze der Kanüle schützen.

Die Erfindung wird im Folgenden anhand mehrerer Figuren beschrieben. Die hierbei offenbarten Merkmale bilden je einzeln und in Kombination die Erfindung vorteilhaft weiter. Es zeigen:
- Figur 1: eine Längsschnittansicht einer ersten Ausführungsform der erfindungsgemässen Vorrichtung mit einer Längsachse (L) in einem Auslieferungszustand.
- Figur 2: einer Längsschnittansicht der Vorrichtung gemäss Figur 1 in einem verabreichten Zustand.
- Figur 3: eine Längsschnittansicht der Vorrichtung gemäss Figur 1 in einem verriegelten Zustand.
- Figur 4: eine Längsschnittansicht einer zweiten Ausführungsform der erfindungsgemässen Vorrichtung mit einer Längsachse (L) in einem Auslieferungszustand.
- Figur 5: einer Längsschnittansicht der Vorrichtung gemäss Figur 4 in einem verabreichten Zustand.
- Figur 6: eine Längsschnittansicht der Vorrichtung gemäss Figur 4 in einem verriegelten Zustand.
- Figur 7: eine Längsschnittansicht einer dritten Ausführungsform der erfindungsgemässen Vorrichtung mit einer Längsachse (L) in einem Auslieferungszustand.
- Figur 8: einer Längsschnittansicht der Vorrichtung gemäss Figur 7 in einem verabreichten Zustand.
- Figur 9: eine Längsschnittansicht der Vorrichtung gemäss Figur 7 in einem verriegelten Zustand.

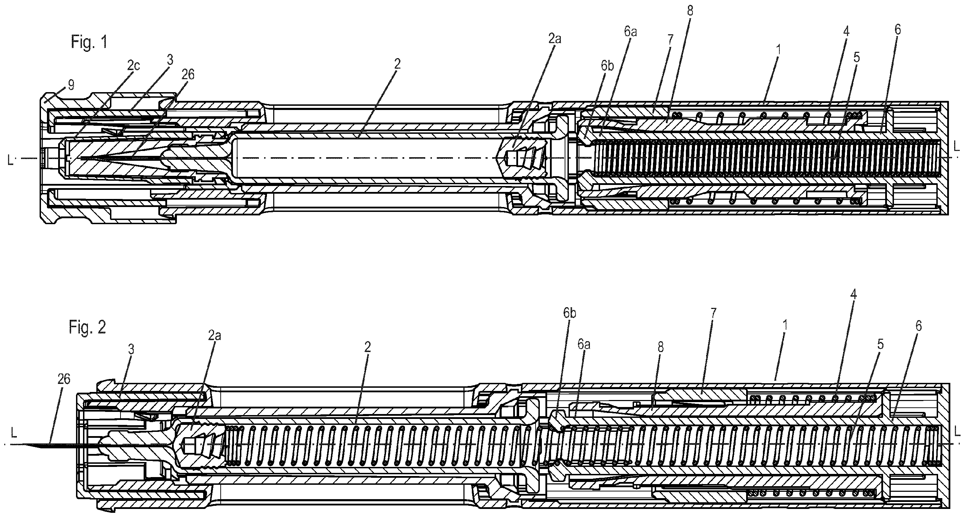

In der Figur 1 ist eine Längsschnittansicht einer ersten Ausführungsform der erfindungsgemässen Vorrichtung in Form eines Autoinjektors mit einer Längsachse (L) in einem Auslieferungszustand ersichtlich. Die Vorrichtung dient der Verabreichung einer fluiden Substanz, insbesondere eines Medikaments. Die Vorrichtung weist ein Gehäuse (1) auf. Das Gehäuse (1) kann hülsenförmig ausgebildet sein. In dem Gehäuse (1) ist ein Produktbehälter (2) mit der zu verabreichenden Substanz axial fest aufgenommen. In dem Produktbehälter (2) ist ein Stopfen (2a) axial versschiebbar aufgenommen, um die Substanz aus dem Produktbehälter (2) zu verabreichen. Die Vorrichtung umfasst eine Nadel (2b), durch welche die Substanz verabreicht werden kann. Die Nadel (2b) ist an dem distalen Ende des Produktbehälters (2) vorgesehen. Des Weiteren umfasst die Vorrichtung eine Nadelschutzelement (3). Das Nadelschutzelement (3) dient zum Schutz vor Stichverletzungen durch die Nadel (2b), insbesondere einer distalen Spitze der Nadel (2b) des Produktbehälters (2). Das Nadelschutzelement (3) kann hülsenförmig ausgebildet sein. Ferner weist die Vorrichtung eine Nadelschutzfeder (4) auf, welche mit dem Nadelschutzelement (3) gekoppelt ist, wobei das Nadelschutzelement (3) von einer distalen Position, in welcher die distale Spitze der Nadel (2b) von dem Nadelschutzelement (3) umgeben ist, in eine proximale Position, in welcher die distale Spitze der Nadel (2b) von dem Nadelschutzelement (3) freigeben ist, axial bewegbar ist. Die Vorrichtung umfasst ferner eine Injektionsfeder (5), welche die Energie zur Bewirkung der Substanzverabreichung zur Verfügung stellt und welche mit dem Stopfen (2a) zusammenwirkt, um die Substanz aus dem Produktbehälter durch die Nadel (2b) zu verabreichen. In dem Auslieferungszustand der Vorrichtung sind die Nadelschutzfeder (4) und die Injektionsfeder (5) als vorgespannte Druckfedern vorgesehen. Die Vorrichtung umfasst zudem ein relativ zu dem Gehäuse (1) axial fest angeordnetes Halteelement (6), in welchem die Injektionsfeder (5), insbesondere vorgespannt und entspannbar aufgenommen ist. Das Halteelement (6) ist zwischen einer Eingriffsposition, in welcher das Halteelement in Eingriff mit der Injektionsfeder (5) ist, und einer Auslöseposition, in welcher das Halteelement (6) ausser Eingriff mit der Injektionsfeder (5) ist, radial nach aussen bewegbar oder radial auslenkbar. Das Halteelement (6) umfasst einen Haltearm (6a). Zudem umfasst der Haltearm (6a) eine Haltenocke (6b). Der Haltearm (6a) ist radial nach aussen bewegbar oder radial auslenkbar. Der Haltearm (6a) des Halteelements (6) erstreckt sich von einem proximalen Ende zu einem distalen Ende, wobei das proximale Ende des Halteelements (6) oder des Haltearms (6a) axial fest an dem Gehäuse (1) angeordnet ist. Der Haltearm (6a) des Halteelements (6) ist elastisch ausgebildet. Die Haltenocke (6b) des Halteelements (6) ist derart ausgebildet, dass die Haltenocke (6b) zwischen der Eingriffsposition, in welcher die Haltenocke (6b) in Eingriff mit der Injektionsfeder (5), insbesondere einer oder den Windungen der Injektionsfeder (5) ist, und einer Auslöseposition, in welcher die Haltenocke (6b) ausser Eingriff mit der Injektionsfeder (5), insbesondere der oder den Windungen der Injektionsfeder (5) ist, radial nach aussen bewegbar oder radial auslenkbar ist. In dem Auslieferungszustand der Vorrichtung hält die Haltenocke (6b) des Halteelements (6) die Injektionsfeder (5) vorgespannt in Eingriff. Die Vorrichtung umfasst ferner eine Schalthülse (7), welche entlang der Längsachse (L) zwischen der Nadelschutzfeder (5) und dem Nadelschutzelement (3) angeordnet ist. Die Schalthülse (7) kann hülsenförmig ausgebildet sein. Die Schalthülse (7) kann von dem Nadelschutzelement (3) in die proximale Richtung mitgenommen werden, wenn das Nadelschutzelement (3) aus der distalen Position in die proximale Position verschoben wird. Die Vorrichtung weist ferner eine Sperrhülse (8) auf, welche quer zur Längsachse (L) zwischen der Schalthülse (7) und dem Haltelement (6) angeordnet ist. Die Sperrhülse (8) kann hülsenförmig ausgebildet sein. Die Sperrhülse (8) ist relativ zu der Schalthülse (7) drehbar angeordnet. Die Schalthülse (7) ist relativ zu dem Gehäuse (1) drehfest angeordnet. Die Sperrhülse (8) kann in der distalen Position des Nadelschutzelements (3) ein radiales nach aussen Bewegen oder ein radiales Auslenken des Halteelements (6) aus dem Eingriff zwischen dem Halteelement (6) und der Injektionsfeder (5) verhindern. Das proximale Ende der Nadelschutzfeder (4) ist an dem proximalen Ende der Sperrhülse (8) abgestützt. Die Sperrhülse (8) ist axial bewegbar auf oder an dem Halteelement (6) angeordnet. In dem Auslieferungszustand der Vorrichtung ist die Sperrhülse (8) in einem lösbaren Eingriff mit dem Haltelement (6). Die Sperrhülse (8) kann einen Vorsprung (nicht ersichtlich) aufweisen, welcher lösbar in Eingriff mit einer Ausnehmung (nicht ersichtlich) an dem Haltelement (6) sein kann.

Damit der Patient die Vorrichtung, insbesondere den Autoinjektor zum Verabreichen der Substanz benutzen kann, muss der Patient vorerst eine an der Vorrichtung lösbar angeordnete Kappe (9) von der Vorrichtung abnehmen. Das Abnehmen der Kappe (7) von der Vorrichtung bewirkt das Wegnehmen einer an der Nadel (2a) lösbar angebrachten Nadelschutzkappe (2c). Danach drückt der Patient die Vorrichtung gegen die Haut, wobei das Nadelschutzelement (3) von der distalen Position in die proximale Position relativ zu dem Gehäuse (1) verschoben wird. Die Nadel (2b) wird dabei in die Haut des Patienten gestochen. Durch diese relative Bewegung des Nadelschutzelements (3) zu dem Gehäuse (1) wird die mit der Nadelschutzfeder (4) beaufschlagte Schalthülse (7) relativ zu dem Gehäuse (1) in die proximale Richtung verschoben. In der proximalen Position des Nadelschutzelements (3) kann die Schalthülse (7) die Sperrhülse (8) freigeben. Die Haltenocke (6b) des Halteelements (6) kann aufgrund der Kraft der Injektionsfeder (5) radial nach aussen bewegbar oder radial auslenkbar sein, sodass die Haltenocke (6b) des Halteelements (6) ausser Eingriff mit der Injektionsfeder (5), insbesondere der oder den Windungen der Injektionsfeder (5) gelangt. Der Vorsprung (nicht ersichtlich) der Sperrhülse (8) gelangt ausser Eingriff mit der Ausnehmung (nicht ersichtlich) des Halteelements (6). Der Vorsprung (nicht ersichtlich) der Sperrhülse (8) bewegt sich radial nach aussen oder ist radial ausgelenkt und kann eine Kulissenführung bilden. Die Kulissenführung des Vorsprungs (nicht ersichtlich) kann in eine an der Schalthülse (7) vorgesehene Kulissenführung eingreift. Durch die beaufschlagende Kraft der Nadelschutzfeder (4) bewegt sich die Sperrhülse (8) axial relativ zu der Schalthülse (7) und zu dem gehäusefesten Halteelement (6) in die proximale Richtung und gelangt in Anschlagkontakt mit dem Halteelement (6), wobei ein Ausschüttstartsignal, insbesondere ein hörbarer Anschlag erzeugt wird. Während der axialen Bewegung der Sperrhülse (8) in die proximale Richtung dreht die Sperrhülse (8) aufgrund der Kulissenführung zwischen dem Vorsprung (nicht ersichtlich) der Sperrhülse (8) und der Schalthülse (7) relativ zu der Schalthülse (7). Durch die relative Bewegung zwischen der Sperrhülse (8) und der Schalthülse (8) gelangt ein an der Sperrhülse (8) vorgesehener Sperranschlag (nicht ersichtlich) in eine axiale Flucht von einem an der Schalthülse (7) vorgesehenem Sperrelement (nicht ersichtlich). In einer alternativen Ausführungsform der erfindungsgemässen Vorrichtung kann die Sperrhülse das Sperrelement und die Schalthülse den Sperranschlag aufweisen.

In der ersten Ausführungsform der erfindungsgemässen Vorrichtung wirkt die Injektionsfeder (5) unmittelbar auf den Stopfen (2a) des Produktbehälters (2). Zudem entspannt sich die Injektionsfeder (5) während der Verabreichung der fluiden Substanz unmittelbar in den Produktbehälter (2). Dadurch kann eine Vorrichtung mit einer hohen Verabreichungsleistung und einer kompakten Bauweise bereitgestellt werden. Während der Verabreichung der fluiden Substanz aus dem Produktbehälter (2) entspannen sich die Windungen der Injektionsfeder (5) relativ zu dem Haltelement (6) und über die Haltenocke (6b) des Haltearms (6a), um ein Ausschüttsignal, insbesondere ein akustisches Ausschüttsignal in Form eines hörbaren Ratterns oder Klickens zu erzeugen. Am Ende der Verabreichung, wie in der Figur 2 gezeigt ist, ist insbesondere das akustische Ausschüttsignal verstummt, wobei dem Patienten angezeigt wird, dass die Verabreichung der fluiden Substanz aus dem Produktbehälter (2) beendet ist und der Patient die Vorrichtung von der Haut wegnehmen kann. Beim Wegnehmen der Vorrichtung von der Haut bewegt sich das Nadelschutzelement (3) und die Schalthülse (7) aufgrund der Kraft der Nadelschutzfeder (4) von der proximalen Position wieder in die distale Position oder in eine von der distalen Position unterschiedliche distale Position.

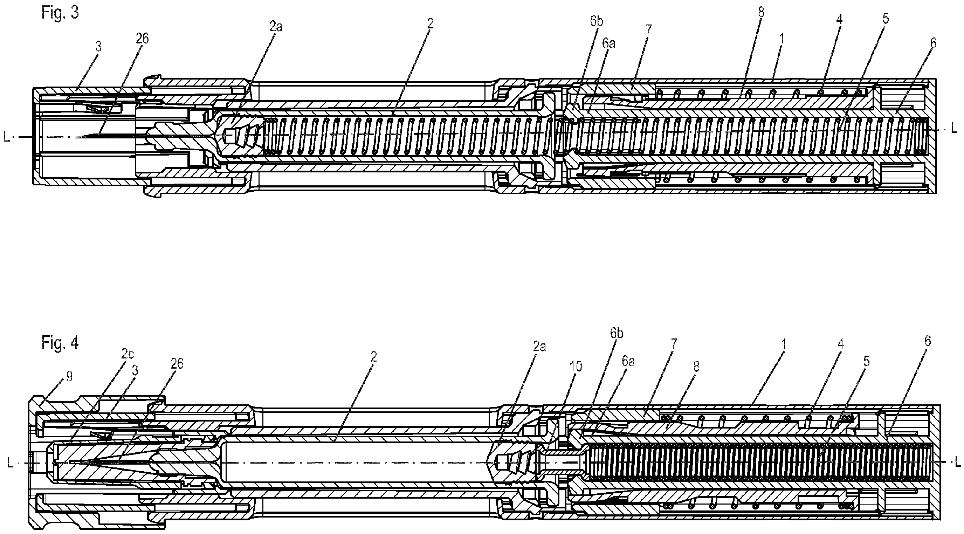

Die Schalthülse (7) weist bevorzugt das Sperrelement (nicht ersichtlich) auf, welches elastisch ausgebildet ist. Das Sperrelement (nicht ersichtlich) kann radial auslenkbar ausgebildet sein. Die Sperrhülse (8) weist bevorzugt den Sperranschlag (nicht ersichtlich) auf. Aufgrund der axialen und rotativen Relativbewegung zwischen der Sperrhülse (8) und der Schalthülse (7) gelangt der Sperranschlag (nicht ersichtlich) der Sperrhülse (8) in die axiale Flucht zu dem Sperrelement (nicht ersichtlich) der Schalthülse (7). Der Sperranschlag (nicht ersichtlich) der Sperrhülse (8) kann bevorzugt rampenförmig mit einem Absatz (nicht ersichtlich) ausgebildet sein, wobei der Absatz (nicht ersichtlich) an einem distalen Ende des Sperranschlags (nicht ersichtlich) angeordnet sein kann. Während der Bewegung des Nadelschutzelements (3) und Schalthülse (7) von der proximalen Position in die distale Position oder in eine von der distalen Position unterschiedliche distale Position, wobei die Schalthülse (7) und das Nadelschutzelement (3) von der Kraft der Nadelschutzfeder (4) in die distale Richtung bewegt werden, gleitet das Sperrelement (nicht ersichtlich) der Schalthülse (7) über den rampenförmigen Sperranschlag (nicht ersichtlich) der Sperrhülse (8). Das Sperrelement (nicht ersichtlich) der Schalthülse (7) kann sich hinter dem Absatz (nicht ersichtlich) des Sperranschlags (nicht ersichtlich) der Sperrhülse (8) entspannen und/oder einschnappen. Bei einer erneuten Bewegung des Nadelschutzelements 83) in die proximale Richtung kann das Sperrelement (nicht ersichtlich), insbesondere das entspannte Sperrelement (nicht ersichtlich) der Schalthülse (7) in Anschlagkontakt mit dem Sperranschlag (nicht ersichtlich), insbesondere mit dem Absatz (nicht ersichtlich) des Sperrelements (nicht ersichtlich) der Sperrhülse (8) gelangen. Somit ist das Nadelschutzelement (3) gesperrt und kann den Patienten vor einer Verletzung durch die Nadel (2), insbesondere durch die distale Spitze der Nadel (2b) schützen. Die Vorrichtung ist in einem verriegelten Zustand, wie in der Figur 3 ersichtlich ist. Alternativ kann der Sperranschlag an der Schalthülse (7) und das Sperrelement an der Sperrhülse (8) vorgesehen sein.

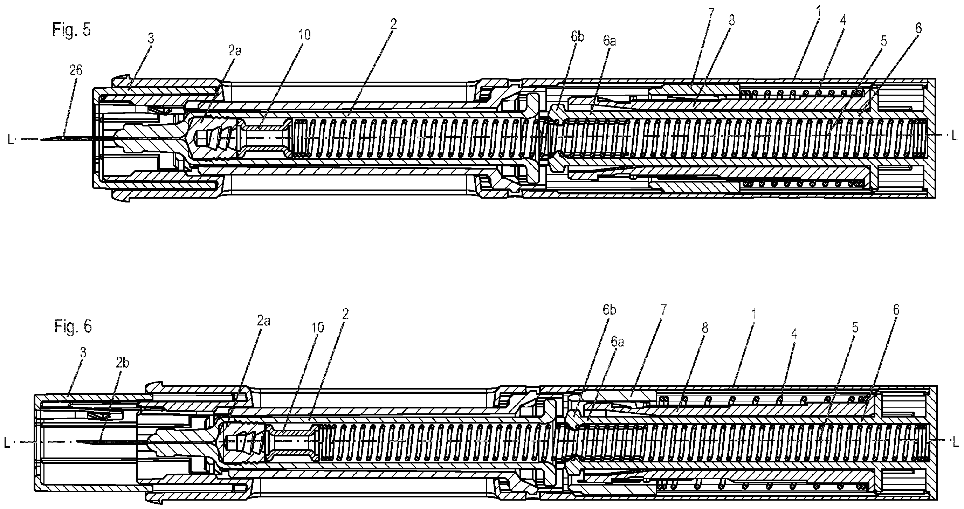

Die Figuren 4, 5 und 6 zeigen eine zweite Ausführungsform der erfindungsgemässen Vorrichtung zum Verabreichen einer fluiden Substanz, wobei die Vorrichtung in der Figur 4 in einem Auslieferungszustand, in der Figur 5 in einem verabreichten Zustand und in der Figur 6 in einem verriegelten Zustand gezeigt ist. Die zweite Ausführungsform unterscheidet sich von der ersten Ausführungsform der erfindungsgemässen Vorrichtung durch das Vorhandensein eines zusätzlichen Adapters (10). Der Adapter (10) ist zwischen der Injektionsfeder (5) und dem Stopfen (2a) des Produktbehälters (2) angeordnet. Die Injektionsfeder (5) wirkt somit über den Adapter (10) auf den Stopfen (2a), um die Substanz zu verabreichen. Der Adapter (10) kann hülsenförmig ausgebildet sein. Somit können verschieden geformte Injektionsfedern (5) und verschieden geformte Stopfen (2a) zusammengebaut werden. Zudem kann der Adapter (10) bevorzugt als Dämpfungselement ausgebildet sein, um die Kraft der Injektionsfeder (5) zu verringern oder entsprechend anzupassen.

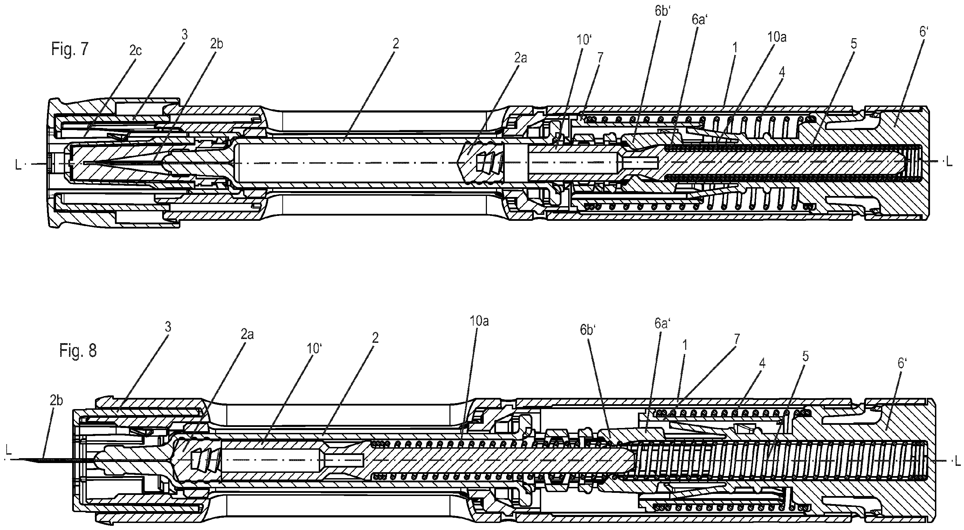

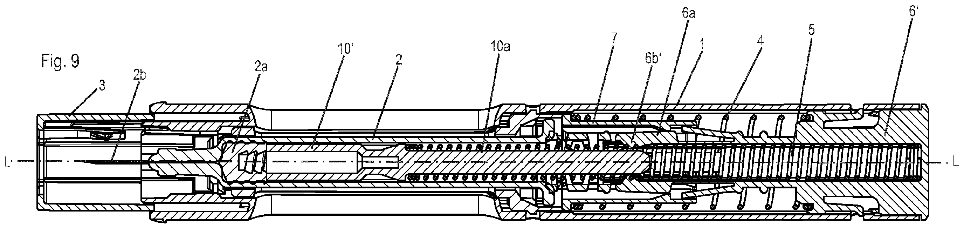

Die Figuren 7, 8 und 9 zeigen eine dritte Ausführungsform der erfindungsgemässen Vorrichtung zum Verabreichen einer fluiden Substanz, wobei die Vorrichtung in der Figur 7 in einem Auslieferungszustand, in der Figur 8 in einem verabreichten Zustand und in der Figur 9 in einem verriegelten Zustand gezeigt ist. Die dritte Ausführungsform unterscheidet sich von der zweiten Ausführungsform der erfindungsgemässen Vorrichtung durch die Gestalt des Halteelements (6') und durch die Gestalt des Adapters (10'). Das Halteelement (6') weist einen ersten Haltearm (nicht ersichtlich') mit einer ersten Haltenocke (nicht ersichtlich) auf, die von einer Eingriffsposition, in welcher die Haltenocke (nicht ersichtlich) in Eingriff mit der oder den Windungen der Injektionsfeder (5) ist, in eine Auslöseposition, in welcher die Haltenocke (nicht ersichtlich) ausser Eingriff mit der oder den Windungen der Injektionsfeder (5) ist, radial nach aussen bewegbar oder radial auslenkbar ist. Das Halteelement (6') weist zudem einen zweiten Haltearm (6a') mit einer zweiten Haltenocke (6b') auf, welche dazu dient, um beim Verabreichen der Substanz ein Ausschüttsignal, insbesondere ein akustisches Ausschüttsignal in Form eines hörbaren Ratterns oder Klickens zu erzeugen. Dieser zweite Haltearm (6a') kann ebenfalls elastisch ausgebildet sein. Es können eine Vielzahl von ersten (nicht ersichtlich) und/oder zweiten Haltearmen (6a') mit den entsprechenden Haltenocken an dem Halteelement (6') vorgesehen sein. Das distale Ende der Injektionsfeder ist an einem distalen Ende des Adapters (10') und das proximale Ende der Injektionsfeder ist an einem proximalen Ende des Halteelements (6') abgestützt. Der Adapter (10') weist eine Führung (10a) auf. Die Führung (10') ist an einer Oberfläche des Adapters (10') angeordnet, sodass bei der Entspannung der Injektionsfeder (5) die Injektionsfeder (5) geführt wird. In einer weiteren Ausführungsform der erfindungsgemässen Vorrichtung kann zusätzlich eine relativ zu dem Gehäuse bewegbare Hülse (nicht ersichtlich) vorgesehen sein, welche über den zweiten Haltearm (6a') mit der zweiten Haltenocke (6b") bewegbar ist, um ein Ausschüttsignal, insbesondere ein akustisches Ausschüttsignal in Form eines hörbaren Ratterns oder Klickens zu erzeugen. Diese Hülse (nicht ersichtlich) kann quer zur Längsachse (L) zwischen der Injektionsfeder (5) und der zweiten Haltenocke (6b") angeordnet sein. Diese Hülse (nicht ersichtlich) kann eine oder mehrere Ausnehmungen und/oder eine oder mehrere Abragungen aufweisen, um das Ausschüttsignal zu erzeugen.

### Bezugszeichen:

1 Gehäuse
2 Produktbehälter
2a Stopfen
2b Nadel
2c Nadelschutzkappe
3 Nadelschutzelement
4 Nadelschutzfeder
5 Injektionsfeder
6; 6' Halteelement
6a Haltearm
6a' zweiter Haltearm
6b Haltenocke
6b' zweite Haltenocke
7 Schalthülse
8 Sperrhülse
9 Kappe
10; 10' Adapter
10a Führung
L Längsachse

## Patentansprüche

1. Vorrichtung zum Verabreichen einer fluiden Substanz mit einer Längsachse (L) umfassend:
1.1 ein Gehäuse (1),
1.2 ein in dem Gehäuse (1) axial fest aufgenommenen Produktbehälter (2) mit der zu verabreichenden Substanz und einem in dem Produktbehälter (2) axial verschiebbar aufgenommenen Stopfen (2a) und einer an dem Produktbehälter vorgesehene Nadel (2b), um die Substanz zu verabreichen;
1.3 ein relativ zu dem Gehäuse (1) verschiebbares Nadelschutzelement (3) für die Nadel (2b);
1.4 eine Nadelschutzfeder (4), welche mit dem Nadelschutzelement (4) gekoppelt ist, wobei das Nadelschutzelement (4) von einer distalen Position, in welcher die distale Spitze der Nadel (2b) von dem Nadelschutzelement (3) umgeben ist, in eine proximale Position, in welcher die distale Spitze der Nadel (2b) von dem Nadelschutzelement freigeben ist, axial bewegbar ist;
1.5 eine Injektionsfeder (5), welche die Energie zur Bewirkung der Substanzverabreichung zur Verfügung stellt und welche mit dem Stopfen (2a) zusammenwirkt, um die Substanz aus dem Produktbehälter (2) durch die Nadel (2b) zu verabreichen;
1.6 ein relativ zu dem Gehäuse (1) axial fest angeordnetes Halteelement (6; 6'), in welchem die Injektionsfeder (5) aufgenommen ist und das Halteelement (6; 6') zwischen einer Eingriffsposition, in welcher das Halteelement (6; 6') in Eingriff mit der Injektionsfeder (5) ist, und einer Auslöseposition, in welcher das Halteelement (6; 6') ausser Eingriff mit der Injektionsfeder ist, radial nach aussen bewegbar oder radial auslenkbar ist;
1.7 **dadurch gekennzeichnet, dass** bei der axialen Bewegung des Nadelschutzelements (3) von der distalen Position in die proximale Position das Halteelement (6; 6') ausser Eingriff mit der Injektionsfeder (5) gelangt, um die Substanz aus dem Produktbehälter (2) durch die Nadel (2b) zu verabreichen.

2. Vorrichtung nach Anspruch 1, **dadurch gekennzeichnet, dass** die Injektionsfeder (5) unmittelbar auf den Stopfen (2a) wirkt, um die Substanz zu verabreichen.

3. Vorrichtung nach Anspruch 1, **dadurch gekennzeichnet, dass** die die Injektionsfeder (5) über einen Adapter (10), der entlang der Längsachse (L) zwischen der Injektionsfeder (5) und dem Stopfen (2a) angeordnet ist, auf den Stopfen (2a) wirkt, um die Substanz zu verabreichen.

4. Vorrichtung nach einem der vorhergehenden Ansprüche, **dadurch gekennzeichnet, dass** die Nadelschutzfeder (4) und/oder die Injektionsfeder (5) als Wendelfeder ausgebildet sind.

5. Vorrichtung nach einem der vorhergehenden Ansprüche, **dadurch gekennzeichnet, dass** beim Verabreichen der Substanz die Injektionsfeder (5), insbesondere die Windungen der Injektionsfeder relativ zu dem Halteelement (6; 6') und über das Halteelement (6; 6') entspannbar sind, um ein akustisches, visuelles und/oder taktiles Ausschüttsignal zu erzeugen.

6. Vorrichtung nach einem der vorhergehenden Ansprüche, **gekennzeichnet durch** eine Schalthülse (7), welche entlang der Längsachse (L) zwischen der Nadelschutzfeder (4) und der Nadelschutzelement (3) angeordnet ist, wobei die Schalthülse (7) von dem Nadelschutzelement (3) in die proximale Richtung mitgenommen wird, wenn das Nadelschutzelement (3) aus der distalen Position in die proximale Position verschoben wird.

7. Vorrichtung nach dem Anspruch 6, **gekennzeichnet durch** eine Sperrhülse (8), welche quer zur Längsachse (L) zwischen der Schalthülse (7) und dem Halteelement (6; 6') angeordnet ist, wobei die Sperrhülse (8) in der distalen Position des Nadelschutzelements (3) ein radiales nach aussen Bewegen oder radiales Auslenken des Halteelements (6; 6') aus dem Eingriff zwischen dem Halteelement (6; 6') mit der Injektionsfeder (5) verhindert.

8. Vorrichtung nach den Ansprüchen 6 und 7, **dadurch gekennzeichnet, dass** die Schalthülse (7) in der proximalen Position des Nadelschutzelements (3) die Sperrhülse (8) freigibt und das Halteelement (6; 6') in die Auslöseposition mit der Injektionsfeder (5) gelangt.

9. Vorrichtung nach den Ansprüchen 6 und 7, **dadurch gekennzeichnet, dass** die Sperrhülse (8) und die Schalthülse (7) eine Kulissenführung einen Sperranschlag und ein korrespondierendes Sperrelement aufweisen, wobei die Sperrhülse (8) und die Schalthülse (7) relativ zueinander axial bewegbar und drehbar angeordnet sind, und wobei während der Bewegung des Nadelschutzelements (3) von der distalen Position in die proximale Position das Sperrelement in eine axiale Flucht mit dem Sperranschlag gelangt, wobei bei einer erneuten Bewegung des Nadelschutzelements (3) in die proximale Richtung das Sperrelement in Anschlagkontakt mit dem Sperranschlag gelangt, um eine erneuten Bewegung des Nadelschutzelements (3) in die proximale Position zu verhindern.

10. Vorrichtung nach einem der Ansprüche 7 bis 9, **dadurch gekennzeichnet, dass** die Sperrhülse (8) relativ zu dem Halteelement (6; 6) in die proximale Richtung bewegbar und in Anschlagkontakt mit dem Halteelement (6; 6) gelangbar ist, um ein akustisches, visuelles und/oder taktiles Ausschüttstartsignal zu erzeugen.
